# EUROPEAN PATENT SPECIFICATION

(11) **EP 2 548 450 B1**
(45) Date of publication and mention of the grant of the patent: **26.04.2017**
(21) Application number: 10847765.4
(22) Date of filing: 18.03.2010
(51) Int. Cl.: A23L 5/30, A23L 3/26, A61Q 5/00, A61Q 19/00, A61K 8/64

(54) **METHOD FOR IMPROVING FUNCTIONAL PROPERTIES BY MEANS OF PULSED LIGHT, SAMPLES WITH IMPROVED FUNCTIONAL PROPERTIES AND USES THEREOF**
VERFAHREN ZUR VERBESSERUNG VON FUNKTIONSEIGENSCHAFTEN MITHILFE VON GEPULSTEM LICHT, PROBEN MIT VERBESSERTEN FUNKTIONSEIGENSCHAFTEN UND VERWENDUNGEN DAVON
PROCÉDÉ POUR AMÉLIORER LES PROPRIÉTÉS FONCTIONNELLES AU MOYEN DE LUMIÈRE PULSÉE, ÉCHANTILLONS AYANT DES PROPRIÉTÉS FONCTIONNELLES AMÉLIORÉES ET UTILISATIONS DE CES DERNIERS

(43) Date of publication of application: 23.01.2013
(73) Proprietor: Fundacion Azti/Azti Fundazioa, 48395 Sukarrieta (Vizcaya) (ES)
(72) Inventor: ARBOLEYA PAYO, Juan, Carlos, E-48395 Sukarrieta (Vizcaya) (ES); ARTÍGUEZ BÁRCENA, Maria, Luz, E-48395 Sukarrieta (Vizcaya) (ES); FERNÁNDEZ PINTO, Estibaliz, E-48395 Sukarrieta (Vizcaya) (ES); MARTÍNEZ DE MARAÑÓN IBABE, Iñigo, E-48395 Sukarrieta (Vizcaya) (ES)
(74) Representative: Carpintero Lopez, Francisco
(86) International application number: PCT/ES2010/070163
(87) International publication number: WO 2011/113968

(56) References cited:
- WO-A1-03/066108
- US-A- 5 034 235
- US-A1- 2009 269 441
- VATANASUCHART N ET AL: "Molecular properties of cassava starch modified with different UV irradiations to enhance baking expansion", CARBOHYDRATE POLYMERS, APPLIED SCIENCE PUBLISHERS, LTD. BARKING, GB, vol. 61, no. 1, 4 July 2005 (2005-07-04), pages 80-87, XP027721697, ISSN: 0144-8617 [retrieved on 2005-07-04]
- DUNN J ET AL: "PULSED-LIGTH TREATMENT OF FOOD AND PACKAGING", FOOD TECHNOLOGY, INSTITUTE OF FOOD TECHNOLOGISTS, CHICAGO, IL, US, vol. 49, no. 9, 1 September 1995 (1995-09-01), pages 95-98, XP000530193, ISSN: 0015-6639
- CHUNG S.-Y. ET AL: 'Effects of pulsed UV-light on peanut allergens in extracts and liquid peanut butter' JOURNAL OF FOOD SCIENCE vol. 73, no. 5, June 2008, ISSN 1750-3841 pages C400 - C404
- ELMNASSER N. ET AL: 'Effect of pulsed-light treatment on milk proteins and lipids' JOURNAL OF AGRICULTURAL AND FOOD CHEMISTRY vol. 56, no. 6, 26 March 2008, ISSN 0021-8561 pages 1984 - 1991

## Description

### FIELD OF THE INVENTION

The invention relates to the field of the compositions with improved properties used in food, cosmetics, biotechnology, biomedicine and pharmacy, among other industrial sectors. In particular, the invention relates to a method for improving the functional properties of samples containing components that can be modified when they absorb ultraviolet light by using pulsed light. Also, the invention relates to the samples with improved functional properties that can be obtained by the same and to the use thereof.

### BACKGROUND OF THE INVENTION

As it is well known in the state of the art, compositions based on macromolecules such as proteins, carbohydrates, lipids, etc. are used in the food industry. Proteins, for example, have different physico-chemical properties: hydration, glass transition, solubility, water retention capacity, oil retention capacity, thickening, dispersing, emulsifying, foaming or gelling properties, film formation properties, bulking agent properties, surface properties (wettability, adhesion,...), among other interesting functional properties. These properties are truly positive for the food industry (preparation of confectionery and bakery products, sauces, meat products, fermented or unfermented dairy products, drinks, etc.).

Also, the functional properties of these macromolecules (proteins and carbohydrates mostly) are applied also in cosmetics with the purpose of obtaining creams, foams and facial or hair gels, shampoos, emulsions, etc., with a particular texture for a better application of the product and subsequent distribution of the active principle of the same or to encapsulate or bind particles, for example.

On the other hand, in the pharmaceutical field is also widely known the use of additives or vehicles of protein type, for example, in the preparation of creams, ointments, emulsions, foams, lotions and other pharmaceutical forms for topical, eye, transdermal application, etc., as well as for obtaining microcapsules, nanocapsules, supporting materials, controlled release systems, etc.

Likewise, compositions based on proteins and carbohydrates have also been used for biomedical and biotechnological applications, particularly for obtaining biomedical materials such as implants, pieces incorporated into the body, suture points or tissue support in which are used biopolymers. Another example of biotechnology application may be the use of these compounds as surfactants (stabilizers of emulsions and foams) for the elimination of ink in recycled paper or for the collection of undesirable or toxic oils by formation of emulsions.

In the case of the protein compositions for the food industry, for example, their functional properties have been improved through texturing methods (by freezing, by extrusion or expansion, or by clotting) or chemical or enzymatic hydrolysis methods, among others.

Thus, for example, Chen et al. proposed a method for improving the foaming properties of a preparation of serum proteins by enzymatic hydrolysis (US 2002/0012720). Baker et al., for their part, defined a method for preparing a whey protein isolate with better foaming properties from an aqueous solution of the same by adjustment of pH at 5-8 and subsequent warming at 60-80 ºC (US 2002/0051843). Likewise, Gustaw et al. described a method for obtaining whey protein isolates gels by the conventional method of water bath and subsequent microwave heating. The gels obtained at pH 3 and 10 by microwave heating had a structure of fine bands, producing stronger gels (improved gelling properties) than those obtained by conventional heating (Gustaw and Mleko 2007 "Gelation of whey proteins by microwave heating", Milchwissenschaft 62 (4): 439-442). Also, Schmitt et al. described a process of preparation of a foodstuff containing native whey protein and that, through heat treatment, at least 20% of these proteins are transformed into micelles which have very good foaming and emulsifying capacity, being able to carry out this heat treatment by microwaves (EP 1839504). Finally, Wilkinson described a method for producing milk foam for the preparation of cappuccino and other beverages based on coffee from a composition comprising milk proteins such as whey. Said composition is emulsified and it is introduced into a spray bottle such that it is obtained a product that is refrigerated and that, subsequently, when dispensed and heated with microwave, is very similar to the milk foam produced by steam (US 2004/0076730).

In the state of the art, therefore, there is still the need for macromolecules compositions (proteins, carbohydrates, lipids, etc.) with improved functional properties for use in the food industry and in other industrial sectors.

Surprisingly, the present inventors have discovered that the application of pulsed light to liquid samples containing proteins allows improving the functional properties of the same.

WO 03/066108 A1 discloses methods and treatment systems for inactivation of microbes and / or nucleic acids in biological fluids, said methods comprising illuminating a platelet composition with one or more pulses of a light. The irradiation of proteins has been proposed by Tada et al. (US 6586037) who developed a method for improving the gelling properties of proteins by irradiation of electron beams using a linear electron accelerator. However, said technology is very different from that of pulsed light because the type of electromagnetic waves is different.

Also, there are different works that test changes in the functional properties of carbohydrates and proteins by the application of ultraviolet light in continuous. The treatment of starch by UV light can lower its viscosity (Bertolini et al. 2001, "Free radical formation in UV- and gamma-irradiated cassava starch", Carbohydrate Polymers, 44 (3): 269-271), increase the water retention capacity and the solubility among others. Said treatment can generate in some cases the formation of crosslinked starch molecules causing a clear improvement in its gelling capacities, for providing texture for example, thereby improving the expansion in bakery and pastry products (Vatanasuchart et al. 2005, "Molecular properties of cassava starch modified with different UV irradiations to enhance baking expansion", Carbohydrate Polymers 61: 80-87). The creation of these conformational changes by application of UV is very beneficial even for the application of these hydrogels in the controlled release of active compounds (mainly food and pharmaceutical) or to increase the biocompatibility in materials used in the biomedicine. In the case of proteins, a positive impact on their functional properties has also been found when being treated by UV light. For example, collagen/gelatin samples processed by UV light can show an improvement in gel strength, a marked reduction in viscosity and significant changes in the enthalpy of fusion (Bhat and Karim 2009 "Ultraviolet irradiation improves gel strength of fish gelatine", Food Chemistry 113: 1160-1164).

While the pulsed light has high content of UV light, there are, however, large differences between the treatment with UV light in continuous and the treatment with pulsed light, mainly due to that pulsed light comprises a broad emission spectrum (190-1100nm) that includes not only the UV range, but also visible and infrared, so the wavelengths emitted in each light pulse other than the UV range can affect in a nonspecific mode the functional properties of the different components of the sample treated. On the other hand, the long exposure time required by the conventional continuous UV sources, in addition to the high intensities employed, cause negative effects on the appearance and the quality of the treated samples, so, a priori, the pulsed light technology is not suitable for the improvement of functional properties of samples intended for food applications.

In addition, Elmnasser et al. carried out a study on the effect of the pulsed light on milk protein and lipids, concluding that the processing of raw milk by means of pulsed light did not cause significant changes in the structure or composition of certain milk proteins such as β-lactoglobulin and α-lactalbumin and, therefore, in the functional properties (Elmnasser et al. 2008, "Effect of Pulsed-Light Treatment on Milk Proteins and Lipids", Journal of Agricultural and Food Chemistry 56: 1984-1991).

S.-Y.Chung et al. in the "Effects of pulsed UV-light on peanut allergens in extracts and liquid peanut butter" (Journal of Food Science; June 2008; Vol.73; no.5; pages C400-C404) carry out a study, in which they investigate the feasibility of using pulse UV light to lower the allergenic properties of peanut extracts and liquid peanut butter. In fact, the pulsed light technology has been used for quite some time for the inactivation of a wide range of micro-organisms. The use of inert gas lamps to generate short intense light pulses as a microbial inactivation method began in Japan at the end of the Decade of the 70s, and it was patented a few years later (Hiramoto 1984 US 445042). In 1988, the company UVERG (Ultraviolet Energy Generators, Oakland, California) worked on the development of systems for the decontamination and disinfection of water, air, and surfaces publishing respective patents and publications (Wekhof 1991," Treatment of contaminated water, air and soil with UV flashlamps", Environmental Progress, 10 (4): 241-247; Wekhof, 1992 US 5144146). In the same year, the company Pure Pulse Technologies® (San Diego, California), subsidiary of Maxwell laboratories, acquired the Hiramoto patent (Wekhof 2000. "Disinfection with flash lamps". PDA Journal of Pharmaceutical Science & Technology 54(3): 264-276; Palmieri and Cacace 2005, "High intensity pulsed light technology". En: Emerging technologies for food processing: 279-306). After various research papers, this company patented a new process of pulsed light called Pure Bright® characterized by its broad emission spectrum (Dunn et al., 1989, US 4871559). Among the applications of this new system the inactivation of microorganisms and enzymes in the surface of food and packaging material were included as well as on air or transparent fluids such as water. Between the years 1991 and 1997, Dunn et al. published patents (Dunn et al. 1991 US 5034235) and papers (Dunn et al. 1995, "Pulsed-Light treatment of food and packaging", Food Technology 49: 95-98; Arrowood et al. 1996, "Disinfection of Cryptosporidium parvum oocysts by pulsed light treatment evaluated in an in vitro cultivation model", Journal of Eukaryot Microbiology 43(5): 88S; Dunn 1996, "Pulsed light and electric field for foods and eggs", Poultry Science 75: 1133-1136; Dunn et al. 1997, "Investigation of pulsed light for terminal sterilization of WFI filled blow/fill/seal polyethylene containers", PDA Journal of Pharmaceutical Science & Technology 51(3): 111-115; Dunn et al. 1997, "Pulsed white light food processing", Cereal Food World 42: 510-515) as a result of the early efforts of this company to promote this new technology. In 1996 the US *Food and Drug Administration* (21CFR179.41) approved the use of pulsed light for use during the production, processing and/or manipulation of food (FDA 1996), promoting greater efforts in the research and development of this technology in the field of the food industry. During the later years great advances have been carried out, and patents and papers have been published by researchers associated with companies that produce and sell pulsed light equipment, as the former WekTec and current SteriBeam Systems in Germany (Wekhof 2000. "Disinfection with flash lamps", PDA Journal of Pharmaceutical Science & Technology 54(3): 264-276, 2001, "Pulsed UV to sterilise packaging and to preserve food staffs". Report to the conference "New technologies: future, today?" Campden & Chorleywood Food Research Association (CCFRA). Gloucestershire, United Kindom, 2003, "Ultra-fast sterilisation by disintegration of microorganisms with intense pulsed UV light". Business briefing: Global surgery: 1-5, 2006, "Six logs sterilization in pulsed UV tunnels from Steribeam". Technology News International), Econos in Japan (Hoshida 2001 JP 2001171623), Xenon Corporation in the USA (Panico and Panico 2006 US 7091495) and Claranor in France (Scotto 2007 WO 023227).

Currently, therefore the use of the technology of pulsed light focuses toward the field of microbial inactivation. Recently, in 2009 Beelman et al. have proposed the use of pulsed light for another very different application such as the increase of the content of vitamin D in mushrooms (US 2009/0269441).

Thus, to date pulsed light technology has not been used or suggested for improving the functional properties of protein and/or carbohydrates liquid samples or of other types of samples that contain other compounds that suffer modifications when they absorb ultraviolet light in continuous.

Therefore, the method of the present invention is an alternative method for improving the functional properties of proteins and other components samples that can be modified upon absorbing ultraviolet light in continuous. Said samples with improved functional properties find application in the food industry, the cosmetics industry, the pharmaceutical industry, the biomedical industry, the biotechnological industry and in other industries (paper, fabrics, cements, inks, detergents, etc.).

On the other hand, the method of the invention, as well as improving the functional properties of the treated sample, also achieves the decontamination of the same.

Various scientific papers have shown that the high intensity of each pulse and the greater power of penetration of pulsed light are responsible for a significantly greater microbial inactivation with regard to the application of UV light in continuous (Dunn et al. 1989 US 4871559; Sharma and Demirci 2003, "Inactivation of Escherichia coli O157:H7 on inoculated alfalfa seeds with pulsed ultraviolet light and response surface modeling", Journal of food Science 68(4): 1448-1453; Krishnamurthy et al. 2004, "Inactivation of Staphylococcus aureus by pulsed UV-light sterilization", Journal of Food Protection 67(5): 1027-1030; Lagunas-Solar et al. 2006, "Development of pulsed UV light processes for surface fungal disinfection of fresh fruits", Journal of Food Protection 69(2): 376-384). On the other hand, pulsed light has other advantages over the conventional UV light systems, among which the reduced treatment time, very suitable for continuous processing lines, as well as the use of Xenon lamps which do not require a warm-up period should be noted. In addition, since it is a non-toxic gas, the potential risks at environmental and health levels that may arise from the use of mercury lamps, standard lamps in the UV processing in continuous, are prevented.

### OBJECT OF THE INVENTION

The present invention, therefore, is intended to provide a method for improving the functional properties of a sample, said method being disclosed in any one of the appended claims 1-8.

Another object of the present invention is the sample with improved functional properties, said sample being disclosed in any one of the appended claims 9-11.

Finally, another object of the invention is the use of said sample with improved functional properties in the food industry, the cosmetics industry, the biotechnological industry, the biomedical industry, the pharmaceutical industry or the chemical industry.

### DESCRIPTION OF THE FIGURES

Figure 1 shows the surface tension based on the concentration of untreated beta-lactoglobulin aqueous solutions and beta-lactoglobulin aqueous solutions treated by the method of the invention at different intensities of treatment (with different number of light pulses).
Figure 2 shows the surface elastic modulus based on the concentration of untreated beta-lactoglobulin aqueous solutions and beta-lactoglobulin aqueous solutions treated by the method of the invention at different intensities of treatment (with different number of light pulses).
Figure 3 shows the foaming capacity based on the concentration of untreated beta-lactoglobulin aqueous solutions and beta-lactoglobulin aqueous solutions treated by the method of the invention at different intensities of treatment (with different number of light pulses).
Figure 4 shows the foaming stability based on the concentration of untreated beta-lactoglobulin aqueous solutions and beta-lactoglobulin aqueous solutions treated by the method of the invention at different intensities of treatment (with different number of light pulses).
Figure 5 shows the maximum elastic modulus of the gel formed from an untreated beta-lactoglobulin aqueous solution and from a beta-lactoglobulin aqueous solution treated by the method of the invention with 10 light pulses.
Figure 6 shows the elastic modulus based on the time and the temperature of the gel formed from an untreated serum protein solution and a serum protein solution treated by the method of the invention with 10 light pulses.
Figure 7 shows the elastic modulus based on the time and the temperature of the gel formed from untreated whey and whey treated by the method of the invention with 10 light pulses.
Figure 8 shows the particle size of the emulsion obtained using an untreated beta-lactoglobulin aqueous solution and a beta-lactoglobulin aqueous solution treated by the method of the invention with 10 light pulses.

### DETAILED DESCRIPTION OF THE INVENTION

The present invention provides a method for improving the functional properties of a sample comprising at least one component which can be modified upon absorbing ultraviolet light in continuous (hereinafter referred to as "the method of the invention") comprising the use of pulsed light with an emission spectrum of 190-1100 nm and a high content of ultraviolet light.

In the context of the invention the expression "functional properties" refers to those physicochemical properties of the component in question, whether it is a biomolecule (protein, carbohydrate, lipid, etc.) or other, affecting the behavior of said components in food systems, among others, during the processing, storage, preparation and consumption. That is, any property, with the exception of nutritional ones, affecting its use. Among said functional properties there can be highlighted, for example, the hydration, glass transition, solubility, water retention capacity, oil retention capacity, thickening, dispersing, emulsifying, foaming or gelling properties, film formation properties, properties as supporting agent or surface properties (wettability, adhesion,...).

In the context of the invention the expression "component which can be modified upon absorbing ultraviolet light", refers to a biomolecule or another component that is able to absorb light in the range of wavelengths of the ultraviolet light and which, as a result, suffers structural modifications.

Also, in the context of the invention the expression "a high content of ultraviolet light" refers to that approximately between 20% and 50% of the total of the pulsed light emitted by the equipment in question corresponds to the UV spectrum.

The processing by means of pulsed light consists in the application of successive flashes or flickers of high intensity light on the product to be treated, each pulse being characterized by its short duration and its broad emission spectrum (usually 190-1100 nm, i.e. from ultraviolet (UV) to near infrared (IR)). In addition to by their broad emission spectrum, the light pulses are also characterized by their short duration, usually ranging between 100 and 350 µs.

As it has been detailed previously, the pulsed light technology has been used for the inactivation of a wide range of micro-organisms (Dunn et al. 1989, US 4871559, Wekhof et al., 1991," Treatment of contaminated water, air and soil with UV flashlamps", Environmental progress 10: 241-243 for example) and for the vitamin D increase in mushrooms (Beelman et al. US 2009/0269441).

It should also be noted that the processing of light pulses is more effective and faster than the application of UV light in continuous for producing the same level of microbial inactivation, being therefore a technology that can be implemented more successfully and easily in continuous processing lines, in particular those of highspeed or productivity or high throughput.

The pulse generator system consists basically of an electric unit, with a capacitor of electrical power and high voltage switches (one per lamp); one or several Xenon lamps; and a control module that, in the case of having several lamps, allows to select between the simultaneous or sequential emission of light pulses.

For the emission of each light pulse, the electrical energy accumulates in the capacitor. Subsequently, the electrical power is magnified when this energy is released very quickly in the Xenon lamp or lamps, the electrical energy becoming a flash of light of high intensity which is emitted in all directions.

Such and as indicated, in the method of the invention the spectrum of the light emitted in each pulse extends from 190 nm to 1100 nm. In any case, the pulsed light that excites the sample will cover the range of wavelengths corresponding to the peak or peaks of absorption of the component or components of the sample. Also, the pulsed light used in the method of the invention has a high content of UV light, of the order of 20% to 50% of the total of the pulsed light emitted by the equipment used corresponds to the UV spectrum. Although there is variability between the different lamps currently available on the market, around 20% of the total emitted light corresponds to the UV-C spectrum (200-280 nm), having a less content in UV-B (280-320 nm) and UV-A (320-400 nm), which constitute about 8% and 12% of the total, respectively.

Depending on the product to be treated and the configuration of the reactor or treatment chamber, one or more reflectors which, normally, consist of parabolic surfaces manufactured based on highly reflective materials can be placed around each lamp. The function of the reflector or reflectors is to redirect the light emitted by the lamps in all directions towards the area of the reactor where the product to be treated is located. Also, the reactor or treatment chamber is built from highly reflective materials, such that the light that does not initially strike on the product to be treated can be reflected by the walls of the chamber until finally reaching said product, thereby increasing the effectiveness of the process.

In the present day there are various companies that produce and sell pulsed light equipment, such as SteriBeam, Claranor, or Xenon Corporation. Tests carried out with different industrial houses of pulsed light equipment allow to affirm that the functioning of different equipments is common, which is why the results of the study are applicable to any pulsed light equipment.

In the method of the invention, the sample is treated with pulsed light with a total fluence equal or higher than the minimum total fluence from which changes in the functional properties are observed, preferably with a total fluence equal to the minimum total fluence from which said modifications are observed.

Total fluence is defined as the product of the fluence received by an object during a light pulse by the number of pulses. In turn, fluence is defined as the energy or amount of photons received per area unit by an object treated with pulsed light during a given exposure time (usually expressed in J/cm²). In the sense of fluence the term dose of light is sometimes used, since it is a more intuitive term, which refers to the energy or amount of photons absorbed per area unit by an object treated with pulsed light during a given exposure time (usually expressed in J/cm²). However it is not a suitable term since the word dose implies complete absorption. Although the invention relates to a method for improving the functional properties of samples containing components that can be modified when they absorb ultraviolet light by using pulsed light, not all the light in the range of 190-1100 nm, notably in the ultraviolet range (190-400 nm), that reaches the components of the sample is absorbed by these.

The maximum total fluence will be that marked by the corresponding legislation. Thus, for example, in the field of food industry, the only existing legislation in this regard is the American legislation (FDA, 21 CFR179.41, 1996), according to which the permitted maximum total fluence is of 12 J/cm² during the use of pulsed light in the production, processing or manipulation of food.

Total fluence is determined by the voltage, radiant energy, radiant power emitted, radiant exitance, fluence speed, pulse fluence, exposure time, distance to the lamps and number of pulses, although it will be enough to provide the values of the total fluence for the skilled in the art to carry out the method of the invention.

In any case the sample to be treated will contain at least one component that is susceptible to modifications upon absorbing ultraviolet light. Thus, in a particular embodiment of the method of the invention, at least one component of the treated sample is a protein, a peptide, a carbohydrate, a lipid, or mixtures thereof.

In the case that the sample comprises several components with different absorption spectra, it may happen that in the treatment with pulsed light some components will be changed positively, while others will be modified in a negative way. Thus, for example, in the case that the sample includes two components with different absorption spectra, an improvement of the functional properties of component "a" can be achieved without changing component "b". To do so, filters to eliminate part of the emitted spectrum can be used such that the spectrum of light reaching the sample has the wavelength that positively modifies component "a" but not the wavelengths that affect component "b" in a negative way. This effect can be achieved, in addition to with a filter that allows the passage of some wavelengths but not others, also with another type of lamp with different emission spectrum i.e. that emits only those wavelengths that positively affect component "a". The method of the invention uses pulsed light, although it would be possible to alternatively use, to reduce the emission spectrum, pulsed laser in the range of wavelengths that positively modify component "a" of the sample without negatively altering component "b" of the sample.

In a particular embodiment, said component is a protein. Said protein can be one or more proteins of animal origin, plant origin, microbial origin or one or more proteins produced through biotechnology, or mixtures thereof. Among proteins of animal origin milk proteins, blood serum proteins, myofibrillar proteins, regulatory proteins, sarcoplasmic proteins, egg proteins, etc. can be mentioned. In a preferred embodiment, said protein is an animal protein. In an even more preferred embodiment, said protein is selected among beta-lactoglobulin, beta-casein, and alpha-lactalbumin.

In another particular embodiment, said component is a mixture of at least one protein and at least one carbohydrate. Said carbohydrate can be one or more carbohydrates selected from monosaccharides, disaccharides and polysaccharides. In a preferred embodiment, said carbohydrate is a disaccharide. In an even more preferred embodiment, said carbohydrate is selected from lactose and sucrose. In another particular embodiment, said component is a mixture of at least one protein and at least one peptide. In another particular embodiment, said component is a mixture of at least one protein and at least one lipid. In another particular embodiment, said component is a mixture of at least one peptide and at least one carbohydrate. In another particular embodiment, said component is a mixture of at least one peptide and at least one lipid. In another particular embodiment, said component is a mixture of at least one carbohydrate and at least one lipid.

These components can be commercial or can be obtained by methods known in the state of the art. Thus, for example, beta-lactoglobulin may be bovine beta-lactoglobulin B ≥90% (PAGE) L8005, marketed by Sigma, or it can be the one obtained by a chromatography separation process from whey.

Also, the sample to be treated in the method of the invention can be solid, liquid, in the form of foam, spray, nebulization, etc. In a particular embodiment of the method of the invention, said sample is a liquid sample. Liquid samples can be, for example, solutions, dispersions, suspensions, emulsions, etc., wherein the type of solvent will be selected by the skilled in the art. In another particular embodiment, the sample is a solid sample. In another particular embodiment, the sample is a nebulized sample wherein the small drops have a high concentration of solid particles. In another particular embodiment, the sample is a spray. In another particular embodiment, the sample is a foam.

In principle, the concentration of the component or components of the sample to be treated by the method of the invention may be any, though the expert will determine the limits of the same. The mixture percentages in any case will be in weight/weight, volume/volume or weight/volume, for example.

In a preferred embodiment, said sample is a liquid sample comprising at least one protein. In an even more preferred embodiment, said liquid sample is a solution of one or more milk proteins such as a solution of beta-lactoglobulin or a solution of serum proteins. In another even more preferred embodiment, said liquid sample is whey, which is a mixture of serum proteins and carbohydrates such as lactose, among other various components.

In the case of liquid samples, the concentration of the component of interest can be very variable. Thus, for example, in the case of a liquid sample comprising one or more proteins, the protein concentration will vary depending on its nature, the type of liquid sample, pH, temperature, etc.

In a particular embodiment of the method of the invention, the liquid sample has a protein concentration of 0.0001 - 1000 mg/ml. In a preferred embodiment, the liquid sample has a protein concentration of 0.001 - 1000 mg/ml. In an even more preferred embodiment, the liquid sample has a protein concentration of 0.01 - 200 mg/ml. Thus, the liquid sample to be treated can be a solution of beta-lactoglobulin with a protein concentration of 0.01-200 mg/ml, preferably of 0.1-100 mg/ml and, more preferably, 0.5-10 mg/ml. Also, the liquid sample to be treated can be a solution of serum proteins with a protein concentration of 0.01-200 mg/ml, preferably of 0.1-100 mg/ml and, more preferably, 0.5-10 mg/ml. Likewise, the liquid sample to be treated can be whey with a protein concentration of 0.01-200 mg/ml, preferably of 0.1-100 mg/ml and, more preferably of 0.5-10 mg/ml.

It is important to highlight that the method of the invention, in addition to improving the functional properties of the treated sample, also allows the decontamination of the same. Thus, the method of the invention entails obtaining a combined effect of improvement of the functionality and recontamination of the sample.

In another aspect, the invention provides a sample with improved functional properties obtainable by the method previously described, wherein the improved functional properties are the properties of hydration, glass transition, solubility, water retention capacity, oil retention capacity, thickening, dispersing, emulsifying, foaming, gelling, film formation, properties as bulking and surface agent such as the wettability and adhesion properties previously described. In a particular embodiment the invention provides a sample with improved functional properties obtainable by the method previously described, wherein the improved functional properties are foaming, gelling or emulsifying properties. This improvement is greater as the intensity of the treatment increases, i.e. as the total fluence applied increases (through the increase in the number of light pulses, for example). Said sample, also presents a reduced or practically non-existent microbiological load and, therefore, better conservation properties.

In any case, the sample will be treated with pulsed light with a total fluence equal to or greater than the minimum required to produce the changes in the functional properties, such and as mentioned previously, preferably with a total fluence equal to the minimum required to produce said modifications. The maximum total fluence of the pulsed light used in the method of the invention will be the one permitted by the legislation, preferably a maximum total fluence of 12 J/cm²which is the maximum limit set by the FDA. These total fluence values can be obtained by different pulsed light equipment, such and as it has been mentioned.

Thus, in a particular embodiment, the sample with improved functional properties is a liquid sample comprising at least one protein with a concentration of 0.0001-1000 mg/ml that has been treated with pulsed light with a total fluence of up to 12 J/cm². In another particular embodiment, the sample with improved functional properties is a liquid sample comprising at least one protein with a concentration of 0.001-1000 mg/ml that has been treated with pulsed light with a total fluence of 0.3-10 J/cm². In a preferred embodiment, the sample with improved functional properties is a liquid sample of at least one protein with a concentration of 0.01-200 mg/ml that has been treated with pulsed light with a total fluence of 0.3-3 J/cm².

In a more preferred embodiment, the sample with improved functional properties is a beta-lactoglobulin solution with a concentration of 0.01-200 mg/ml that has been treated with pulsed light with a total fluence of 0.3-3 J/cm². In another more preferred embodiment, it is a solution of serum proteins with a concentration of 0.01-200 mg/ml that has been treated with pulsed light with a total fluence of 0.3-3 J/cm². In another more preferred embodiment, said liquid sample is whey with a protein concentration of 0.01-200 mg/ml that has been treated with pulsed light with a total fluence of 0.3-3 J/cm².

In an even more preferred embodiment, the sample with improved functional properties is a beta-lactoglobulin solution with a concentration of 0.1-100 mg/ml that has been treated with pulsed light with a total fluence of 0.3-3 J/cm². In another even more preferred embodiment, it is a solution of serum proteins with a concentration of 0.1-100 mg/ml that has been treated with pulsed light with a total fluence of 0.3-3 J/cm². In another even more preferred embodiment, said liquid sample is whey with a protein concentration of 0.1-100 mg/ml that has been treated with pulsed light with a total fluence of 0.3-3 J/cm².

In an even more preferred embodiment, the sample with improved functional properties is a beta-lactoglobulin solution with a concentration of 0.5-10 mg/ml that has been treated with pulsed light with a total fluence of 0.3-3 J/cm². In another more preferred embodiment, it is a solution of serum proteins with a concentration of 0.5-10 mg/ml that has been treated with pulsed light with a total fluence of 0.3-3 J/cm². In another more preferred embodiment, said liquid sample is whey with a protein concentration of 0.5-10 mg/ml that has been treated with pulsed light with a total fluence of 0.3-3 J/cm².

In another aspect, the invention also provides the use of the sample with improved functional properties previously described in different applications of the food industry, the cosmetics industry, the pharmaceutical industry, the biomedical industry, the biotechnological industry, the chemical industry as well as in other industries such as the industry of paper, fabrics, cements, inks, detergents or the environmental management (collection of undesirable oils and elimination of ink, for example). Thus, in a particular embodiment, the sample with improved functional properties previously described is used in the food industry, the cosmetics industry, the biotechnological industry, the biomedical industry, the pharmaceutical industry or the chemical industry.

The following examples illustrate the invention and must not be construed as limiting the scope of the same.

The conditions of treatment used in the examples were the following:
- Pulsed light equipment: XeMaticA-(L+L), SteriBeam. The results obtained in tests conducted with another pulsed light equipment from another trading house, Claranor, allow to state that the improvements of the functional properties are available with any pulsed light equipment.
- Treatment conditions: 2.2 kV/pulse, 300 J per pulse, 0.3 J/cm²/pulse, 6.5 cm distance to the lamp. Duration of each pulse 300 µs, approximately. Treatments of 0.3 to 3 J/cm² (1 p to 10p) (The results in the graphics appear depending on the number of pulses).
- The liquid samples were treated by introducing a volume of the same in a treatment reactor that allows the passage of emitted light.

### EXAMPLE 1

### Treatment of beta-lactoglobulin aqueous solutions for improving their properties of surface tension, surface rheology, foaming capacity, foaming stability and gelling properties.

Different amounts of a commercial beta-lactoglobulin sample were dissolved (L-8005 Sigma, bovine beta-lactoglobulin B ≥90% (PAGE)) in ultrapure water with constant surface tension (72.5 mN/m) for obtaining different solutions with protein concentrations of 0.1 to 10 mg/ml.

These solutions were treated with 4 or 10 light pulses, according to the treatment conditions previously indicated using as a control an untreated sample.

### 1.1 Measurement of surface tension

The surface tension of each of these solutions was measured through the pendant drop technique, using a pulsating drop tensiometer FTA200 (First Ten Angstroms, USA). This technique measures the surface tension over time through the analysis of the image of a drop pending from a syringe. The shape of the drop (determined by its density and surface tension) is analyzed using the capillarity equation to finally obtain the value of surface tension in a given time. The volume of the syringe was of 100 µl and the initial drop had a volume of 12 µl. The needle of the syringe had a diameter of 0.94 mm. All the measurements were performed at room temperature (approximately 20 ºC). Used protein concentrations: 0.1-1.5 mg/ml

### Results

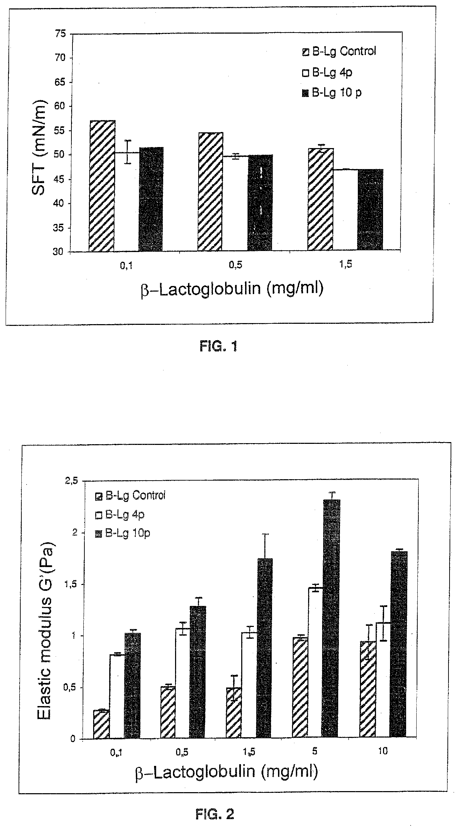

**Figure 1** shows the surface tension depending on the concentration of the untreated beta-lactoglobulin solution (Control B-Lg) and the beta-lactoglobulin solution treated by the method of the invention at different intensities of treatment: with 4 pulses (B-Lg 4 p) and with 10 pulses (B-Lg 10p).

As it can be observed, the surface tension values were better for the treated solution than for the untreated solution. Moreover, when the protein concentration in the solution was higher greater differences between the untreated and the treated solution were observed. On the other hand, the surface tension values were clearly lower as the protein solution was treated with greater intensity of light, and there is no big difference between the treatment with 4 pulses and the treatment with 10 pulses.

It is noteworthy that the fact that a solution has lower surface tension means that the protein will be more adsorbed at the air-water interface and, therefore, it will have a better foaming capacity.

### 1.2 Measurement of the surface rheology

The surface rheology experiments were carried out by using an AR2000 Advanced Rheometer (TA Instruments) rheometer. An aluminum bicone with a diameter of 60 mm and a cone angle of 4:59:13 was used. This geometry was placed in the water-air interface. The oscillatory measures were carried out with a frequency of 1 Hz. all the measurements were carried out in the viscoelastic linear region with a deformation value of 0.014. The values of the elastic modulus (G') of the interface were recorded for 30 minutes. The values represented in the different solutions are those achieved at minute 30. Used protein concentrations: 0.1-10 mg/ml.

### Results

**Figure 2** shows the surface elastic modulus depending on the concentration of untreated beta-lactoglobulin aqueous solutions (B-Lg Control) and beta-lactoglobulin aqueous solutions treated by the method of the invention at different intensities of treatment (with different number of light pulses): with 4 pulses (B-Lg 4p) and with 10 pulses (B-Lg 10p).

The surface elastic modulus provides a measure of the stability of a formed foam: the greater the elasticity in the interface is, the greater the resistance to the drainage of a foam created thus allowing for greater foam stability.

As it can be seen, there are differences for all the tested concentrations. Also, there are large differences between the untreated sample and the one treated with 4 pulses and with 10 pulses, the change being higher with the more intense treatment.

### 1.3 - Measurement of the foaming properties: foaming capacity and foaming stability

The measures of foaming capacity and stability were carried out in a Foamscan Instrument (France). With this instrument, said values are determined through conductivity measures. The foam is generated by the application of a nitrogen flow of 500 ml/min and is passed through a porous glass filter for 30 seconds (pore diameter 10-16 µm). The foaming capacity was determined by the difference between the total volume of the formed foam and the initial volume of the solution (30 ml). The foaming stability was determined by the mean time (t_{(1/2)} - amount of solution drained at half measurement time). Used protein concentrations: 0.5-10 mg/ml

### Results

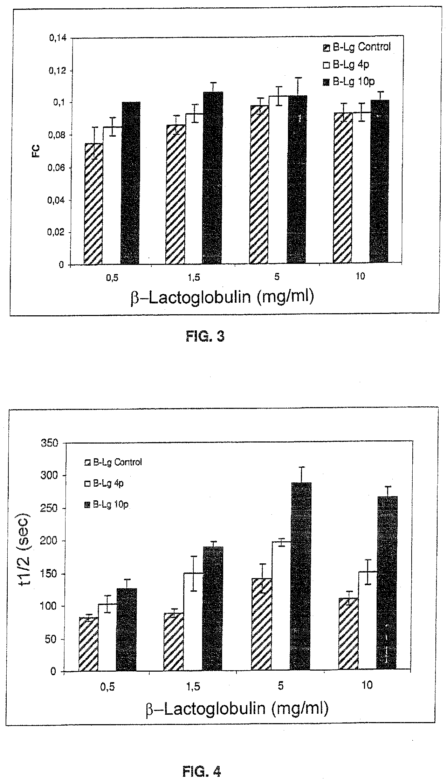

**Figure 3** shows the foaming capacity (FC) depending on the concentration of untreated beta-lactoglobulin aqueous solutions (B-Lg Control) and beta-lactoglobulin aqueous solutions treated by the method of the invention at different intensities of treatment (with different number of light pulses): with 4 pulses (B-Lg 4p) and with 10 pulses (B-Lg 10p).

The untreated protein had less foaming capacity while the greater the intensity of treatment applied was, said capacity was greater. The main differences can be observed at concentrations of 0.5 and 1.5 mg/ml, although these are not very obvious.

**Figure 4** shows the foaming stability depending on the concentration of untreated beta-lactoglobulin aqueous solutions (B-Lg Control) and beta-lactoglobulin aqueous solutions treated by the method of the invention at different intensities of treatment (with different number of light pulses): with 4 pulses (B-Lg 4p) and with 10 pulses (B-Lg 10p).

The foaming stability achieved with a treated solution was much greater than that achieved with the native protein. The differences among the proteins treated at different light pulses were very significant, and it was clearly observed that the foaming stability achieved with a solution treated at 10 pulses was much greater than the one achieved with the native protein. On the other hand, the effects on the functionality began to be observed from the application of 4 light pulses.

### 1.4 - Measurement of the gelling properties

The values of elastic modulus (G') of the gel formed from a beta-lactoglobulin solution (10 weight/volume, 100 mg/ml) were determined through the use of a AR2000 Advanced Rheometer (TA Instruments) rheometer. A plate-plate steel geometry, 40 mm diameter was used. The oscillatory measures were carried out with a frequency of 1 Hz. All the measurements were made in the viscoelastic linear region with a value of deformation of 0.5%. A peltier plate controlled the temperature during the measurements. The applied temperature profile was the following: an increase in temperature of 20-90 ºC with an increase of 2.5 ºC/min; the temperature was kept at 90 ºC for half an hour and finally a decrease in temperature at 20 ºC was carried out with a decline of 4.5 ºC/min.

### Results

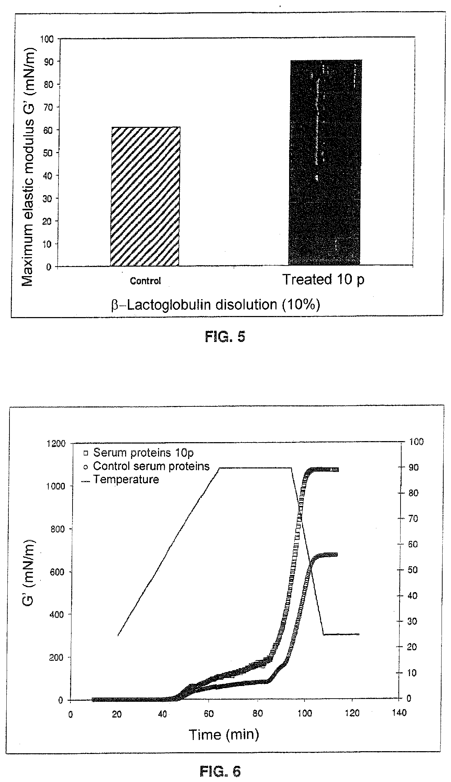

**Figure 5** shows the maximum elastic modulus of the gel formed from an untreated beta-lactoglobulin aqueous solution and from a beta-lactoglobulin aqueous solution treated by the method of the invention with 10 light pulses.

The higher the maximum elastic modulus value is the gelling capacity is higher since there is an increase of the viscoelasticity of the formed network and therefore the strength of said gel.

### EXAMPLE 2

### Treatment of a serum proteins solution for improving its gelling properties.

A sample of commercial serum proteins PROVON 190 - Glanbia Nutritionals (Ireland) (92% serum proteins) was dissolved in ultrapure water with constant surface tension (72.5 mN/m) to obtain a serum proteins solution with a protein concentration of 100 mg/ml.

This solution was treated with 10 light pulses, according to the treatment conditions indicated previously using an untreated sample as a control.

### 2.1 Measurement of the gelling properties

The values of elastic modulus (G') of the gel formed from a serum proteins solution (10% weight/volume, 100 mg/ml) were determined through the use of an AR2000 Advanced Rheometer (TA Instruments) rheometer. A plate-plate steel geometry, 40 mm diameter was used. The oscillatory measures were carried out with a frequency of 1 Hz. All the measurements were made in the viscoelastic linear region with a value of deformation of 0.5%. A peltier plate controlled the temperature during the measurements. The applied temperature profile was the following: an increase in temperature of 20-90 ºC with an increase of 2.5 ºC/min; the temperature was kept at 90 ºC for half an hour and finally a decrease in temperature at 20 ºC was carried out with a decline of 4.5 ºC/min.

### Results

**Figure 6** shows the elastic modulus as a function of time and the temperature of the gel formed from an untreated serum proteins solution (control) and from a serum proteins solution treated by the method of the invention with 10 light pulses.

Figure 6 shows a heating curve of the sample of serum proteins: from 60 ºC the protein without treatment begins a denaturation that causes the gelling phenomenon reaching maximum values of elasticity of the gel of 600 mN/m. On the other hand, the treatment with 10 light pulses shows an effect on the gelling thereof, indicating that the effect of the light on the denaturation of the protein has a positive effect, since the treated protein has elasticity values higher than the untreated protein of up to 1000 mN/m.

In general terms it can be concluded that the application of pulsed light (10 light pulses) improved the properties of the gel formed from this solution of serum proteins.

### EXAMPLE 3

### Treatment of whey for improving its gelling properties.

A whey sample obtained in a cheese shop from cheese elaboration was obtained, and it had the following composition:

| Fat% | Protein % | Humidity % | Dry extract % | Ashes % | Calcium mg/kg | Lactose % |
|---|---|---|---|---|---|---|
| 0.8 | 0.99 | 93.84 | 6.16 | 0.43 | 338 | 3.81 |

This whey was treated with 10 light pulses, according to the treatment conditions previously indicated using an untreated sample as a control.

### 3.1 Measurement of the gelling properties

The values of elastic modulus (G') of the gel formed from whey (direct waste with direct protein content of 1%) were determined through the use of an AR2000 Advanced Rheometer (TA Instruments) rheometer. A plate-plate steel geometry, 40 mm diameter was used. The oscillatory measures were carried out with a frequency of 1 Hz. All the measurements were made in the viscoelastic linear region with a value of deformation of 0.5%. A peltier plate controlled the temperature during the measurements. The applied temperature profile was the following: an increase in temperature of 20-90 ºC with an increase of 2.5 ºC/min; the temperature was kept at 90 ºC for half an hour and finally a decrease in temperature at 20 ºC was carried out with a decline of 4.5 ºC/min.

### Results

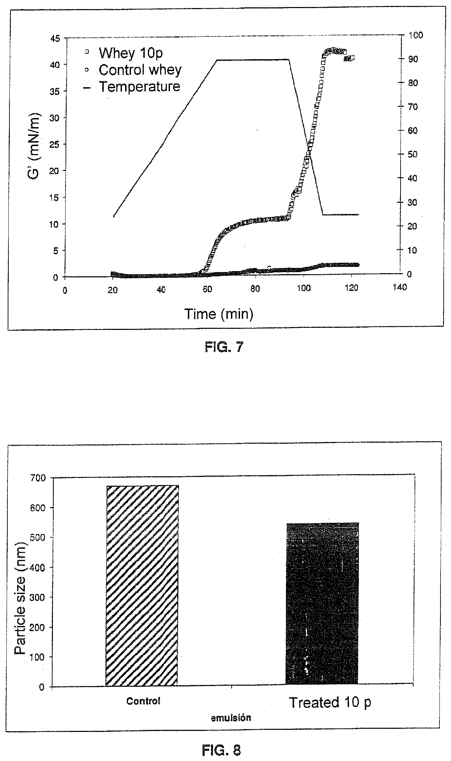

**Figure 7** shows the elastic modulus as a function of time and the temperature of the gel formed from an untreated whey (control) and from whey treated by the method of the invention with 10 light pulses.

Figure 7 shows a heating curve of the sample of serum proteins: from 60 ºC the protein without treatment begins a denaturation that causes the gelling phenomenon reaching very low maximum values of elasticity of the gel (of about 2 mN/m). On the other hand, the treatment with 10 light pulses shows an effect on the gelling thereof, indicating that the effect of the light on the denaturation of the protein has a positive effect, since the treated protein has elasticity values higher than the untreated protein of up to 42 mN/m.

It could be concluded that the application of pulsed light (10 light pulses) clearly improved the properties of the gel formed from whey.

### EXAMPLE 4

### Treatment of a beta-lactoglobulin aqueous solution and for improving its emulsifying properties.

A sample of a commercial beta-lactoglobulin sample was dissolved (L-8005 Sigma, bovine beta-lactoglobulin B ≥90% (PAGE)) in ultrapure water with constant surface tension (72.5 mN/m) for obtaining a beta-lactoglobulin solution with a protein concentration of 1.5 mg/ml.

This solution was treated with 10 light pulses, according to the treatment conditions indicated previously using an untreated sample as a control.

Each of these aqueous solutions, treated and untreated, was used to prepare an oil-in-water emulsion. The oil-in-water emulsions were prepared using 75% of aqueous phase and 25% of lipid phase (n-tetradecane). A pre-homogenization was carried out by vigorous agitation and thereafter, the homogenization was carried out by ultrasound. The emulsifying capacity was determined by the measurement of the particle size. For this, a Malvern Mastersizer nano series was used by measuring the average particle size (d₃₂) by the Light-scattering technique.

### Results

**Figure 8** shows the particle size of the emulsion obtained using an untreated beta-lactoglobulin aqueous solution and a beta-lactoglobulin aqueous solution and treated by the method of the invention with 10 light pulses.

A smaller particle size in the emulsion means that said system has a greater emulsifying stability. The differences between the emulsions measured at time 0 and time 2.25 hours are virtually non-existent.

However, there are significant differences between preparing an emulsion with a sample of protein previously treated by light pulses and preparing it with a sample of untreated protein, concluding that the first has greater emulsifying capacity.

## Claims

1. A method for improving the functional properties of a sample comprising a protein, a peptide, a carbohydrate, a lipid or mixtures thereof which can be modified upon absorbing ultraviolet light, **characterized by** comprising the use of pulsed light with an emission spectrum of 190-1100 nm, wherein from 20% to 50% of the pulsed light emitted corresponds to the UV spectrum and wherein the functional properties are selected from: hydration, glass transition, solubility, water retention capacity, oil retention capacity, thickening, dispersing, emulsifying, foaming, gelling film formation, and surface properties such as wettability or adhesion.

2. Method according to claim 1, **characterized in that** said component of the sample is a protein.

3. Method according to claim 2, **characterized in that** said component of the sample is a protein of animal origin.

4. Method according to claim 3, **characterized in that** said component of the sample is a protein selected from beta-lactoglobulin, beta-casein, and alpha-lactalbumin.

5. Method according to claim 1, **characterized in that** said sample is a liquid sample.

6. Method according to claim 5, **characterized in that** said sample is a liquid sample comprising at least one protein.

7. Method according to claim 6, **characterized in that** the liquid sample has a protein concentration of 0.0001-1000 mg/ml.

8. Method according to claim 7, **characterized in that** the liquid sample has a protein concentration of 0.01-200 mg/ml.

9. Sample obtainable by the method according to claims 1-8.

10. Sample obtainable by the method according to claim 7, **characterized in that** it is a liquid sample of at least one protein with a concentration of 0.0001-1000 mg/ml that has been treated with pulsed light with a total fluence of up to 12 J/cm².

11. Sample obtainable by the method according to claim 8, **characterized in that** it is a liquid sample of at least one protein with a concentration of 0.01-200 mg/ml that has been treated with pulsed light with a total fluence of 0.3-3 J/cm².

12. Use of a sample with improved functional properties according to claims 9-10 in the food industry, the cosmetics industry, the biotechnological industry, the biomedical industry, the pharmaceutical industry or the chemical industry.

## Patentansprüche

1. Verfahren zur Verbesserung der funktionellen Eigenschaften einer Probe, umfassend ein Protein, ein Peptid, ein Kohlenhydrat, ein Lipid oder Gemische davon, welches modifiziert werden kann bei Absorption von ultraviolettem Licht, **gekennzeichnet durch** das Umfassen der Verwendung von gepulstem Licht mit einem Emissionsspektrum von 190-1100 nm, wobei von 20% bis 50% des emittierten gepulsten Lichts dem UV-Spektrum entspricht und wobei die funktionalen Eigenschaften ausgewählt sind aus: Hydratisierung, Glasübergang, Löslichkeit, Wasserrückhaltevermögen, Ölrückhaltevermögen, Verdicken, Dispergieren, Emulgieren, Schäumen, gelierende Filmbildung und Oberflächeneigenschaften wie z.B. Benetzbarkeit oder Adhäsion.

2. Verfahren nach Anspruch 1, **dadurch gekennzeichnet, dass** die Komponente der Probe ein Protein ist.

3. Verfahren nach Anspruch 2, **dadurch gekennzeichnet, dass** die Komponente der Probe ein Protein tierischen Ursprungs ist.

4. Verfahren nach Anspruch 3, **dadurch gekennzeichnet, dass** die Komponente der Probe ein Protein ist, dass ausgewählt ist aus Beta-Lactoglobulin, Beta-Casein und Alpha-Lactalbumin.

5. Verfahren nach Anspruch 1, **dadurch gekennzeichnet, dass** die Probe eine flüssige Probe ist.

6. Verfahren nach Anspruch 5, **dadurch gekennzeichnet, dass** die Probe eine flüssige Probe ist, die mindestens ein Protein umfasst.

7. Verfahren nach Anspruch 6, **dadurch gekennzeichnet, dass** die flüssige Probe eine Proteinkonzentration von 0,0001-1000 mg/ml aufweist.

8. Verfahren nach Anspruch 7, **dadurch gekennzeichnet, dass** die flüssige Probe eine Proteinkonzentration von 0,01-200 mg/ml aufweist.

9. Probe, die durch das Verfahren nach den Ansprüchen 1 bis 8 erhältlich ist.

10. Probe, die erhältlich ist durch das Verfahren nach Anspruch 7, **dadurch gekennzeichnet, dass** sie eine flüssige Probe ist von mindestens einem Protein mit einer Konzentration von 0,0001-1000 mg/ml, das mit pulsiertem Licht mit einer Gesamtfluenz von bis zu 12 J/cm² behandelt worden ist.

11. Probe, die erhältlich ist durch das Verfahren nach Anspruch 8, **dadurch gekennzeichnet, dass** sie eine flüssige Probe ist von mindestens einem Protein mit einer Konzentration von 0,01-200 mg/ml, das mit pulsiertem Licht mit einer Gesamtfluenz von bis zu 0,3-3 J/cm² behandelt worden ist.

12. Verwendung einer Probe mit verbesserten funktionellen Eigenschaften gemäß den Ansprüchen 9 bis 10, in der Lebensmittelindustrie, der Kosmetikindustrie, der biotechnologischen Industrie, der biomedizinischen Industrie, der pharmazeutischen Industrie oder der chemischen Industrie.

## Revendications

1. Procédé d'amélioration des propriétés fonctionnelles d'un échantillon comprenant une protéine, un peptide, un glucide, un lipide ou des mélanges de ceux-ci qui peuvent être modifiées par absorption de lumière ultraviolette, **caractérisé par le fait qu'**il comprend l'utilisation d'une lumière pulsée présentant un spectre d'émission de 190 à 1 100 nm, dans lequel de 20 % à 50 % de la lumière pulsée émise correspondent au spectre UV et dans lequel les propriétés fonctionnelles sont choisies parmi : l'hydratation, la transition vitreuse, la solubilité, la capacité de rétention de l'eau, la capacité de rétention de l'huile, l'épaississement, la dispersion, la mise en émulsion, l'expansion, la formation d'un film par gélification, et les propriétés de surface telles que la mouillabilité ou l'adhérence.

2. Procédé selon la revendication 1, **caractérisé en ce que** ledit composant de l'échantillon est une protéine.

3. Procédé selon la revendication 2, **caractérisé en ce que** ledit composant de l'échantillon est une protéine d'origine animale.

4. Procédé selon la revendication 3, **caractérisé en ce que** ledit composant de l'échantillon est une protéine choisie parmi la bêta-lactoglobuline, la bêta-caséine et l'alpha-lactalbumine.

5. Procédé selon la revendication 1, **caractérisé en ce que** ledit échantillon est un échantillon liquide.

6. Procédé selon la revendication 5, **caractérisé en ce que** ledit échantillon est un échantillon liquide comprenant au moins une protéine.

7. Procédé selon la revendication 6, **caractérisé en ce que** l'échantillon liquide a une concentration de protéine de 0,0001 à 1 000 mg/ml.

8. Procédé selon la revendication 7, **caractérisé en ce que** l'échantillon liquide a une concentration de protéine de 0,01 à 200 mg/ml.

9. Échantillon pouvant être obtenu par le procédé selon les revendications 1 à 8.

10. Échantillon pouvant être obtenu par le procédé selon la revendication 7, **caractérisé en ce qu'**il s'agit d'un échantillon liquide d'au moins une protéine ayant une concentration de 0,0001 à 1 000 mg/ml qui a été traitée par une lumière pulsée présentant une fluence totale allant jusqu'à 12 J/cm².

11. Échantillon pouvant être obtenu par le procédé selon la revendication 8, **caractérisé en ce qu'**il s'agit d'un échantillon liquide d'au moins une protéine ayant une concentration de 0,01 à 200 mg/ml qui a été traitée par une lumière pulsée présentant une fluence totale de 0,3 à 3 J/cm².

12. Utilisation d'un échantillon ayant des propriétés fonctionnelles améliorées selon les revendications 9 à 10 dans l'industrie alimentaire, l'industrie cosmétique, l'industrie biotechnologique, l'industrie biomédicale, l'industrie pharmaceutique ou l'industrie chimique.
